# EUROPEAN PATENT SPECIFICATION

(11) **EP 3 159 343 B1**
(45) Date of publication and mention of the grant of the patent: **19.07.2017**
(21) Application number: 15191125.2
(22) Date of filing: 22.10.2015
(51) Int. Cl.: C07D 487/04, A61K 31/4985, A61P 3/10

(54) **IMPROVED PROCESS FOR THE PREPARATION OF TRIAZOLE AND SALT THEREOF**
VERBESSERTES VERFAHREN ZUR HERSTELLUNG VON TRIAZOLE UND DEREN SALZEN
PROCÉDÉ AMÉLIORÉ POUR LA PRÉPARATION DE TRIAZOLE ET DE SON SEL

(43) Date of publication of application: 26.04.2017
(73) Proprietor: F.I.S.- Fabbrica Italiana Sintetici S.p.A., 36075 Alte di Montecchio Maggiore (VI) (IT)
(72) Inventor: Galvagni, Marco, 37131 Verona (IT); Grendele, Ennio, 36078 Valdagno (VI) (IT); Lora, Giovanni, 36073 Cornado Vicentino (VI) (IT)
(74) Representative: Leganza, Alessandro

(56) References cited:
- WO-A1-03/004498
- WO-A1-2004/085378
- WO-A2-2004/080958
- WO-A2-2009/085990
- JAUME BALSELLS ET AL: "Synthesis of [1,2,4]Triazolo[4,3-[alpha]]piperazines via Highly Reactive Chloromethyloxadiazoles", ORGANIC LETTERS, vol. 7, no. 6, 17 February 2005 (2005-02-17), pages 1039-1042, XP055234040, US ISSN: 1523-7060, DOI: 10.1021/ol0474406

## Description

### Technical Field

The present invention relates to an improved process for the preparation of Triazole and salt thereof, a key intermediate for the synthesis of Sitagliptin.

### Background Art

The compound Triazole, having the following chemical formula (II):

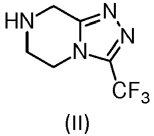

has chemical name 3-(trifluoromethyl)-5,6,7,8-tetrahydro[1,2,4]triazolo[4,3-a]pyrazine and CAS RN 486460-21-3.

Triazole is a key intermediate for the synthesis of Sitagliptin of formula (I): 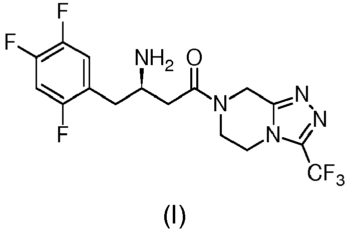 a substance also known as MK-0431 and having chemical name 7-[(3R)-3-amino-1-oxo-4-(2,4,5-trifluorophenyl)butyl]-5,6,7,8-tetrahydro-3-(trifluoromethyl)-1,2,4-triazolo[4,3-a]pyrazine. Sitagliptin is commercially available as the phosphate salt monohydrate under the trade name Januvia.

This active pharmaceutical ingredient is an oral antihyperglycemic (antidiabetic drug) of the dipeptidyl peptidase-4 (DPP-4) inhibitor. This enzyme-inhibiting drug is used alone or in combination with other oral antihyperglycemic agents, such as for example metformin or a thiazolidinedione, for treatment of diabetes mellitus type 2. The benefit of this medicine is its fewer side effects (e.g., less hypoglycemia, less weight gain) in the control of blood glucose values.

A synthetic process for the preparation of Sitagliptin is carried out by means of various steps, in particular one of them is the process for the preparation of Triazole.

A few methods for the preparation of Triazole have been disclosed.

In particular, Organic Letters, 7 (6), 1039-1042, 2005 discloses the synthesis of [1,2,4]Triazolo[4,3-r]piperazines via condensation of high reactive chloromethyloxadiazoles with ethylenediamins. The above mentioned article describes the preparation of different products, such as Oxadiazole, Triazole and substituted Triazoles. In particular, scheme 3, that is present in said article at page 1040, shows and briefly illustrates the preparation of Triazole 1a, starting from oxadiazole 3a proceeding to amidine 4a, thus to obtain 1a, through reflux of 4a in methanol. The experimental details, related to the preparation of 3-(trifluoromethyl)-5,6,7,8-tetrahydro[1,2,4]triazolo [4,3-a]pyrazine hydrochloride salt 1a, are not described in the same article, but they are disclosed in supporting information for [1,2,4]Triazolo[4,3-r]piperazines via condensation of high reactive chloromethyloxadiazoles at page 4.

Specifically, WO2004/080958 discloses, in the example 1, in particular Step D, the preparation of 3-(trifluoromethyl)-5,6,7,8-tetrahydro[1,2,4]triazolo [4,3-a]pyrazine, hydrochloride salt (1-4), that was carried out by means of several steps with different solvents, and mostly with a substantial large amount of volumes, about 15 volumes. In a similar manner the compound is prepared also in WO2009/085990 and WO2004/085378.

In the above mentioned preparation, a warmed suspension of amidine in methanol (4V) was reacted with aqueous hydrochloric acid (37% HCl, 0,4V), producing a seed bed formed after the cooling. Later starting from the beforehand produced mixture, MTBE (11V) was added. The resulting slurry was cooled and filtered. Finally, for obtaining triazole 1-4, the solid cake was washed with ethanol:MTBE (1:3) solution (2V) and dried under vacuum at 45°C. In this method the conversion of amidine to triazole is carried out in methanol and water, while the isolation of the product is carried out from a mixture of methanol, water and MTBE.

The prior art methods this suffers of the drawbacks that the productivity of triazole is relatively low, especially considering the large volumes of solvents used, thus effecting the overall production capacity. Moreover, considering large productions, the recovery of the solvents is quite complex and require long cycling time.

### Summary of invention

The problem addressed by the present invention, in the light of the prior art methods, is therefore that of providing an improved process for the preparation of Triazole and salts thereof which allows to increase the productivity and to avoid the needs of using different solvents, both for performing the conversion reaction and for isolating the product.

This problem is solved by a process for the preparation of Triazole and salts thereof as outlined in the annexed claims, whose definitions are integral part of the present description.

Particularly, the present invention provides a method for producing of Triazole of formula (II): 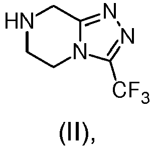 a key intermediate in the synthesis process of active pharmaceutical ingredient Sitagliptin.

Said method comprises the conversion in acid environment of Amidine of formula (III): 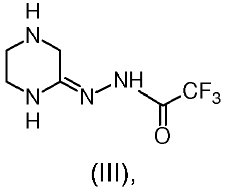 to compound of formula (II), wherein the conversion is carried out in ethanol, only; from thus obtained suspension of compound (II) in ethanol, the product of formula (II) is isolated.

Further features and advantages of the process according to the invention will result from the description hereafter reported of examples of realization of the invention, provided as an indication of the invention.

Described is also a pharmaceutical composition comprising Sitagliptin or salt thereof, obtained by means of the Triazole prepared according process of the present invention, and one or more pharmaceutically acceptable excipients.

### Description of embodiments

Object of the present invention is a process for the preparation of Triazole of formula (II) and salt thereof: 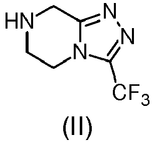 by means of the conversion in acid environment of Amidine of formula (III): 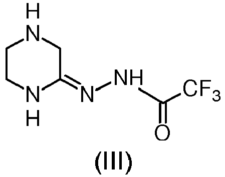 to compound of formula (II), wherein the conversion is carried out in ethanol, thus succeeding in lowering the amount of volumes used to carry out the conversions and to carry out the isolation of the product and, at the same time, avoiding the use of different solvents both in the conversion reaction and isolation of the product.
The produced suspension of compound (II) in ethanol indeed does not comprise any other solvent. Finally, the isolation of the product of formula (II) from the suspension of said compound is carried out in ethanol.

It has been indeed surprisingly found that the conversion in acid environment of Amidine of formula (III) to Triazole of formula (II) and salt thereof by means of ethanol allows a remarkable decrease of the volumes of solvent employed, thus dramatically improving the productivity of the process. The above mentioned reduction is performed using ethanol solvent instead of a mixture of methanol and water, as disclosed in prior art. Furthermore, such type of solvent, therefore employing ethanol rather than methanol and water, allows to carry out the process for the preparation of Triazole without the further addition of methyl tert-butyl ether (MTBE), as in the prior methods. The process described in prior art comprises the addition of MTBE for isolating Triazole, instead, the here present invention allows to produce Triazole without the use of MTBE or the other solvents, using ethanol, only. Therefore, according to beforehand said, the present invention describes a process for the preparation of Triazole (II) and salt thereof using only one solvent, which is ethanol, both for converting (III) to (II) and for isolating (II).

The lower of volumes of solvent used and the fact that only ethanol is used, produces the reduction of wastes. Moreover, said features of the process of the invention allow strong increase of productivity and, at same time, a reduction production costs.

The present invention is a process for the preparation of Triazole of formula (II) and salt thereof: 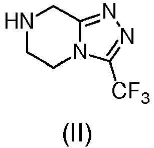 comprising the following steps:
a) conversion in acid environment of Amidine of formula (III): 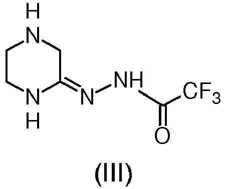 to compound of formula (II), wherein the conversion is carried out in ethanol,
b) obtaining suspension of compound (II) in ethanol,
c) isolation of the product of formula (II) from a suspension of said compound in ethanol.

According to the preferred embodiment of the present invention, the conversion of step a) is carried out in acid environment, specially an solution of gaseous hydrochloric acid (HCl) on denatured ethyl alcohol is dosed to a mixture of Amidine of formula (III) and ethanol.

Besides, according to the above mentioned, the said conversion is carried out in ethanol, this solvent is added to an amount of Amidine of formula (III) to produce a mixture of Amidine in ethanol.

The said mixture of Amidine and ethanol is heated at a temperature comprised between 55°C and 65°C, preferably at a temperature about 60°C.

The heated mixture is dosed whit a solution of gaseous hydrochloric acid on denatured ethanol to convert Amidine of formula (III) to Triazole (II), and thus to produce a suspension of the compound of formula (II).

Said suspension or slurry is an amount of solid material of the compound of formula (II) suspended in ethanol.

The process of the invention is carried out using ethanol as solvent, therefore the suspension of the product of formula (II) is in ethanol, only.

The suspension of compound (II) of the step b) is obtained in ethanol, in particular said suspension is carried out without the addition of any other solvent.

In the step a) is previously prepared a suspension of the compound of formula (III) in ethanol which is then heated at 55-65°C, after the addition of the acid, then said mixture is heated at reflux, in particular it is warmed at temperature about 79°C.

The mixture is kept at about 79°C for at least 30 minutes.

Then the resulting slurry is cooled at room temperature, purposely at temperature between from 20°C to 25°C.

Therefore, according to above described, the suspension of compound (II) of the step b) is obtained in ethanol, after stirring, without the addition whatever solvent, specifically without the addition of methyl tert-butyl ether (MTBE), as instead disclosed in prior art.

In the step b) of the process for the preparation of Triazole of formula (II) and salt thereof of the present invention, the suspension of compound (II) in ethanol is obtained without a the addition of any other solvent.

According to a preferred embodiment of the process of the present invention, the step a) and/or the step b) is carried out without the addition of water.

Specifically, according to a more preferred embodiment of the process of the presence invention, the step a) is carried out without the addition of water or the step b) is carried out without the addition of water or both the steps a) and b) are carried out without the addition of water.

According to an other preferred embodiment of the process of the present invention, the step a) or the step a) and step b), ethanol contains an amount of water lower than 0.10 volumes compared to the compound of formula (III); more preferably ethanol contains an amount of water about 0.05 volumes compared to the same compound of formula (III).

As for definition, 1 volume of solvent is an amount of volume per amount of substance. For example 1 volume can be 1 Liter per 1 Kilogram or 1 mL per 1 gram, 1 microliter per 1 microgram, etc..

According to a preferred embodiment of the process of the present invention, wherein the both steps a) and b) are carried out at an amount of volumes of ethanol between about 1 and about 6 volumes compared to the compound of formula (III).

According to a more preferred embodiment of the process of the present invention, wherein the both steps a) and b) are carried out at an amount of volumes of ethanol between about 1.5 and about 2.0 volumes compared to the compound of formula (III), more preferably said steps are carried out at about 1.7 volumes of ethanol.

The process of the invention provides Triazole with molar yield between from about 89.00% to 90.70%, and a HPLC purity more than 99.7% A/A%.

Finally, starting from the suspension of the compound of the step b) in ethanol, in the step c), Triazole of formula (II) and salt thereof is isolated, by means of a filtration or centrifugation, preferably by filtration, and then the cake is washed with ethanol and then the solid is discharged and dryed under vacuum at a temperature about 50°C.

The process of the presence invention allows the preparation of the following Triazole of formula (II): 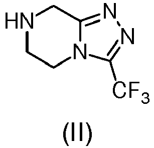 and salt thereof, for example, sulphate, hydrobromide, nitrate salt, etc., preferably the hydrochloride salt having the following structure:

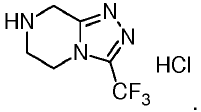

Another aspect of the present invention is the use of ethanol for the conversion of Amidine of formula (III): 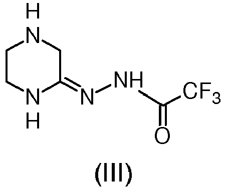 to Triazole of formula (II) and salt thereof: 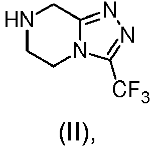 thus obtaining suspension of compound (II) in ethanol.

In particular, said conversion is carried out in ethanol, as beforehand described, the mixture, made up an amount of Amidine of formula (III) in ethanol, reacts in presence of gaseous hydrochloric acid to obtain a suspension of compound (II) and salt thereof, in ethanol.

A further aspect of the invention is the process for the preparation of Sitagliptin of formula (I) and salt thereof: 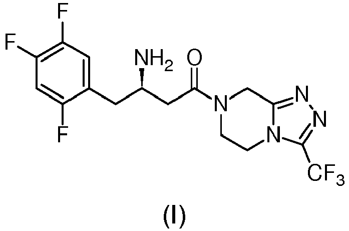 consisting of the following steps:
A) conversion of Amidine of the formula (III) to Triazole of formula (II) and salt thereof according the process of the present invention,
B) conversion of Triazole of the step A) to obtain Sitagliptin.
   The conversion of the step A) have been described above in the paragraphs describing the process for preparation of Triazole of formula (II) and salt thereof.
   Starting from the same Triazole of formula (II), by means of conversion of the step B), Sitagliptin of formula (I) and salt thereof are obtained, since the product (II) is a key intermediate in the process for the synthesis of the same Sitagliptin.
   According to a preferred embodiment of the invention, the process for the preparation of Sitagliptin of formula (I) and salt thereof, wherein the step B) is carried out by means of the following steps:
C) the conversion of Triazole to Ketoamide of formula (IV) 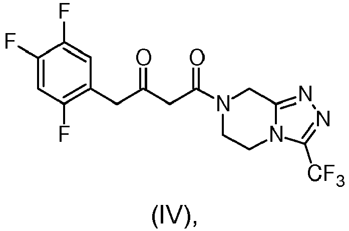
D) the reaction to produce Enamine Amide of formula (V) from Ketoamide, produced in the step C) 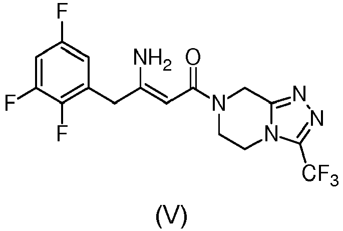
E) the conversion of Enamine Amide of step D) to Sitagliptin of formula (I): 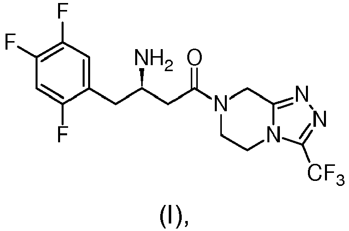 or by means the alternative process for obtaining Sitagliptin, wherein the steps C), D) and E) are substituted by the following steps:
C1) the conversion of Triazole to Ketoamide of formula (IV): 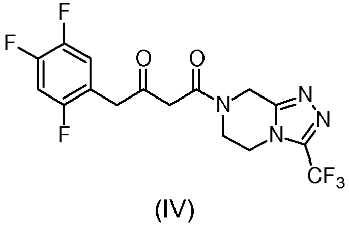
D1) the enzymatic conversion of Ketoamide of formula (IV), obtained by previous step C1), to Sitagliptin of formula (I):

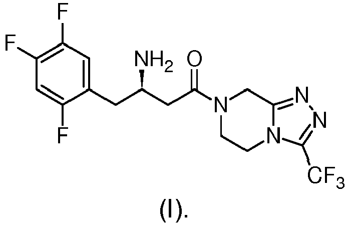

In particular, Ketoamide of formula (IV) of the step C) and the same Ketoamide of the step C1) are produced according to the known prior art methods, such as those disclosed in WO2005/020920, purposely in the Step A, related to scheme 2 of the example 1.

Then, following the process, the reaction to produce Enamine Amide of formula (V) from Ketoamide, produced in the step C) and the conversion of Enamine Amide of step D) to Sitagliptin of formula (I) are disclosed respectively in Step B and in Step C, related to scheme 2, of WO2007/050485.

The alternative process for obtaining Sitagliptin, starting from Ketoamide of formula (IV), beforehand described, consists in the enzymatic conversion of same Ketoamide, obtained by previous step C1), to Sitagliptin of formula (I). This enzymatic conversion is disclosed in the known prior art, in particular in the article of Science, 2010, volume 329, pag. 305-309.

Sitagliptin of formula (I) or salt thereof, prepared according to the above process, may be included in pharmaceutical compositions, comprising one or more pharmaceutically acceptable excipients or in combination with other active pharmaceutical ingredients and one or more pharmaceutically acceptable excipients.

Example of suitable pharmaceutical composition, in particular in combination with other active pharmaceutical ingredients, is a tablet comprising 50 mg of Sitagliptin (as phosphate monohydrate salt) and 850 mg of metformin hydrochloride.

Other examples suitable pharmaceutical compositions are following described:
- 25 mg tablet contains sitagliptin phosphate monohydrate, equivalent to 25 mg sitagliptin,
- 50 mg tablet contains sitagliptin phosphate monohydrate, equivalent to 50 mg sitagliptin,
- 100 mg tablet contains sitagliptin phosphate monohydrate, equivalent to 100 mg sitagliptin,
wherein the above pharmaceutical compositions contain the following excipients in tablet core: microcrystalline cellulose (E460), calcium hydrogen phosphate, anhydrous (E341), croscarmellose sodium (E468), magnesium stearate (E470b) and sodium stearyl fumarate.

Furthermore the film coating of the said pharmaceutical compositions can be made up the following excipients: poly(vinyl alcohol), macrogol 3350, talc (E553b), titanium dioxide (E171), red iron oxide (E172) and yellow iron oxide (E172).

Sitagliptin of formula (I) or salt thereof, prepared according to the above process, or pharmaceutical compositions comprising Sitagliptin alone or combination with other active pharmaceutical ingredients and one or more pharmaceutically acceptable excipients, can be suitable for use in medicine.

Specifically, Sitagliptin of formula (I) or salt thereof, prepared according to the above process, or pharmaceutical compositions comprising Sitagliptin alone or combination with other active pharmaceutical ingredients and one or more pharmaceutically acceptable excipients, can be suitable for use in the treatment of diabetes mellitus type 2.

The skilled in the art of organic chemistry can appreciate as the process of the invention allows a dramatic improvement of the productivity considering the strong reductions of volumes employed to carry out both conversion and isolation of the product from a maximum volumes of about 15 volumes to about 1.7 volumes, at the same time, avoiding the use of solvent mixtures.

### EXPERIMENTAL SECTION

The starting material Amidine can be produced according to the known prior art methods, such as those disclosed in WO2005/020920 or WO2005/097733 or WO2007/050485.

### Example1: Preparation of Triazole of formula (II) as HCl salt in 5 volumes of ethanol with the presence catalytic amount of water.

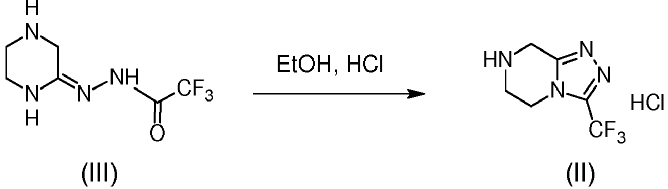

Into a three necked round bottom flask, under nitrogen atmosphere, 250 g of Amidine of formula (III), 1000 mL of denatured ethanol (4V) and 12.5 mL of water (0.05V). The obtained suspension is heated at temperature 60°C and stirring at the same temperature at least 15 min. Then a solution of 45 g HCl (gas) in 250 mL denatured ethanol (1V) was dosed in 20 min., keeping the temperature at temperature range from 55°C to 60°C. The resulting mixture was stirred for 1 hour at the same temperature. The slurry was cooled at room temperature and was stirred for least 30 min. at the same temperature. The obtained slurry was filtered and the solid was washed with 250 mL of denatured ethanol. Drying the solid under vacuum at T=45°C, 246.25 g of Triazole of formula (II) as HCl salt were obtained with 90,53% of molar yield.

### Example 2: Preparation of Triazole of formula (II) as HCl salt in 1.7 volumes of ethanol.

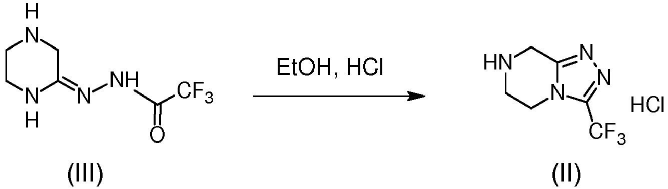

Into a three necked round bottom flask, under nitrogen atmosphere, 500 g of Amidine of formula (III), 450 mL of denatured ethanol (0.9V). The obtained suspension is heated at temperature 60°C and stirring at the same temperature at least 15 min. Then a solution of 90 g HCl (gas) in 315 mL denatured ethanol (0.8V) was dosed in 20 min., keeping the temperature at temperature range from 55°C to 60°C. The resulting mixture was stirred for 1 hour at the same temperature. The slurry was cooled at room temperature and was stirred for least 30 min. at the same temperature. The obtained slurry was filtered and the solid was washed with 500 mL of denatured ethanol. Drying the solid under vacuum at T=45°C, 493.5 g of Triazole of formula (II) as HCl salt were obtained with 90.71 % of molar yield.

### Example 3: Preparation of Triazole of formula (II) as HCl salt in 1.7 volumes of ethanol.

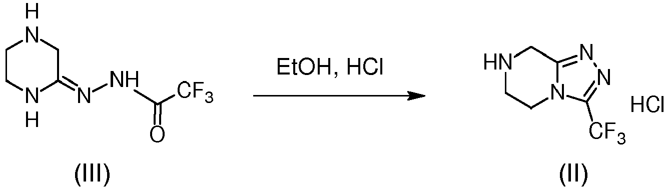

Into a three necked round bottom flask, under nitrogen atmosphere, 180 g of Amidine of formula (III) and 180 mL of denatured ethanol (1V). The obtained suspension is heated at temperature 60°C and stirring at the same temperature at least 15 min. 122,1 g of a solution of HCl (gas) on denatured ethanol (0.7V) were dosed for 20 min., keeping the temperature at temperature range from 60°C to 70°C. The resulting mixture was heated a reflux (T=79°C) for 30 min. The slurry was cooled at room temperature and was stirred for least 30 min. at the same temperature. The obtained slurry was filtered washing the solid with 180 mL of denatured ethanol. Drying the solid under vacuum at T=50°C, 175,7 g of Triazole of formula (II) as HCl salt were obtained with 89,94% of molar yield and HPLC purity of 99.8% A/A%.

### Example 4: Preparation of Triazole of formula (II) as HCl salt in 1.7 volumes of ethanol.

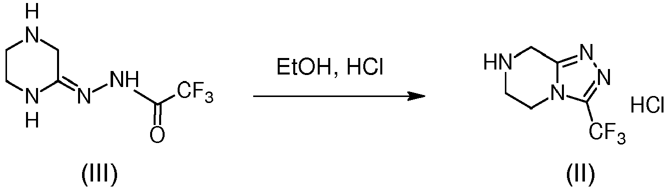

Into a three necked round bottom flask, under nitrogen atmosphere, 180 g of Amidine of formula (III) and 180 mL of denatured ethanol (1V). The obtained suspension is heated at temperature 60°C and stirring at the same temperature at least 15 min. 122,1 g of a solution of HCl (gas) on denatured ethanol (0.7V) were dosed for 20 min., keeping the temperature in a range from 60°C to 70°C. The resulting mixture was heated a reflux (T=79°C) for 30 min. The slurry was cooled at room temperature and was stirred for least 30 min. at the same temperature. The obtained slurry was filtered washing the solid with 180 mL of denatured ethanol. Drying the solid under vacuum at T=50°C, 176,6 g of Triazole of formula (II) as HCl salt were obtained with 90,20% of molar yield. and HPLC purity of 99.8% A/A%.

## Claims

1. Process for the preparation of Triazole of formula (II) and salt thereof: 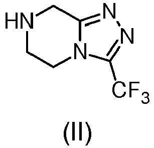 comprising the following steps:
a) conversion in acid environment of Amidine of formula (III): 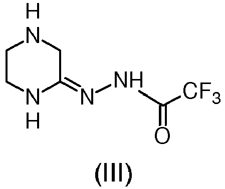 to compound of formula (II), wherein the conversion is carried out in ethanol,
b) obtaining suspension of compound (II) in ethanol, without the addition of any other solvent,
c) isolation of the product of formula (II) from a suspension of said compound in ethanol.

2. Process according to claim 1, wherein the conversion of the step a) is carried out dosing a solution of gaseous hydrochloric acid on denatured ethyl alcohol to a mixture of Amidine of formula (III) and ethanol.

3. Process according to anyone of the claims from 1 to 2, wherein the step a) and/or step b), is carried out without the presence of water.

4. Process according to anyone of the claims from 1 to 2, wherein in the step a) or in both the steps step a) and step b), ethanol contains an amount of water lower than 0.10 volumes compared to the compound of formula (III).

5. Process according to anyone of the claims from 1 to 4, wherein the both steps a) and b) are carried out with an amount of volumes of ethanol comprised between about 1 and about 6 compared to the compound of formula (III).

6. Process according to anyone of the claims from 1 to 5, wherein the volumes of ethanol are comprised between from 1.5 to 2.0 volumes compared to the compound of formula (III).

7. Process according to anyone of the claims from 1 to 6, wherein the volumes of ethanol are 1.7 volumes compared to the compound of formula (III).

8. Process according to anyone of the claims from 1 to 7, wherein the Triazole of formula (II) is in form of hydrochloride salt.

9. Process according to anyone of the claims from 1 to 8, wherein the conversion of step a) is carried out by means of gaseous hydrochloric acid.

10. Use of ethanol for the conversion of Amidine of formula (III): 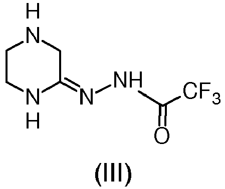 to Triazole of formula (II) and salt thereof: 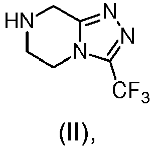 thus obtaining suspension of compound (II) in ethanol.

11. Process for the preparation of Sitagliptin of formula (I) and salt thereof: 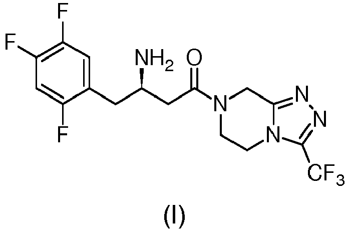 consisting of the following steps:
A) conversion of Amidine to Triazole according to the process of anyone of the claims from 1 to 9,
B) conversion of Triazole of the step A) to obtain Sitagliptin.

12. Process according to claim 11, wherein the step B) is carried out by means of the following steps:
C) the conversion of Triazole to Ketoamide of formula (IV): 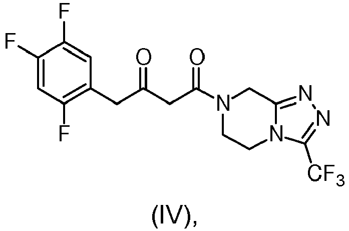
D) reaction for producing Enamine Amide of formula (V) from Ketoamide, produced in the step C) 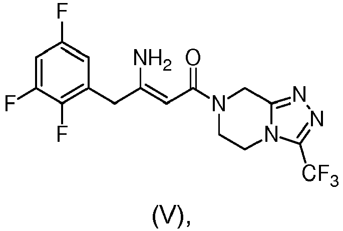
E) the conversion of of Enamine Amide of step D) to Sitagliptin of formula (I): 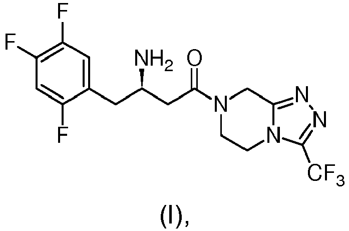 or by means the alternative method for obtaining Sitagliptine consisting the following steps:
C1) the conversion of Triazole to Ketoamide of formula (IV): 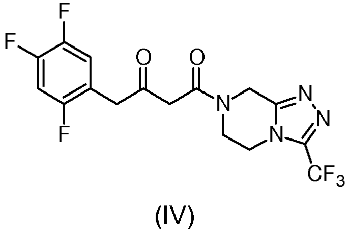
D1) the enzymatic conversion of Ketoamide of formula (IV), obtained by previous step C1), to Sitagliptin of formula (I):

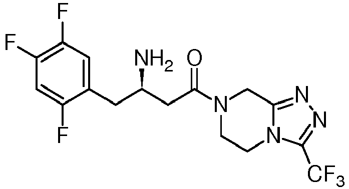

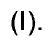

## Patentansprüche

1. Verfahren zur Herstellung von Triazol mit der Formel (II) und Salzen davon: 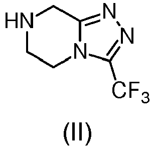 umfassend die folgenden Schritte:
a) Umwandlung von Amidin mit der Formel (III) in saurer Umgebung: 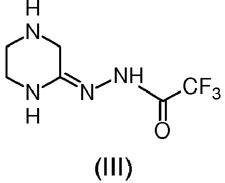 in eine Verbindung der Formel (II), wobei die Umwandlung in Ethanol durchgeführt wird,
b) Erhalten einer Suspension der Verbindung (II) in Ethanol ohne die Zugabe irgendeines anderen Lösungsmittels,
c) Isolierung des Produkts mit der Formel (II) aus einer Suspension der Verbindung in Ethanol.

2. Verfahren nach Anspruch 1, wobei die Umwandlung der Stufe a) durchgeführt wird, indem eine Lösung von gasförmiger Salzsäure in denaturiertem Ethylalkohol zu einer Mischung von Amidin mit der Formel (III) und Ethanol dosiert wird.

3. Verfahren nach einem der Ansprüche 1 bis 2, wobei die Stufe a) und/oder Stufe b) ohne Anwesenheit von Wasser durchgeführt wird.

4. Verfahren nach einem der Ansprüche 1 bis 2, wobei in Stufe a) oder in beiden Stufen, Stufe a) und Stufe b), Ethanol eine Wassermenge unter 0.10 Volumina enthält, verglichen mit der Verbindung der Formel (III).

5. Verfahren nach einem der Ansprüche 1 bis 4, wobei sowohl Stufe a) als auch Stufe b) mit einer Menge der Volumina an Ethanol durchgeführt werden, die zwischen etwa 1 und etwa 6 liegt, verglichen mit der Verbindung der Formel (III).

6. Verfahren nach einem der Ansprüche 1 bis 5, wobei die Volumina an Ethanol zwischen 1.5 und 2.0 Volumen liegen, verglichen mit der Verbindung der Formel (III).

7. Verfahren nach einem der Ansprüche 1 bis 6, wobei die Volumina an Ethanol 1.7 Volumen betragen, verglichen mit der Verbindung der Formel (III).

8. Verfahren nach einem der Ansprüche 1 bis 7, wobei das Triazol mit der Formel (II) in Form des Hydrochloridsalzes vorliegt.

9. Verfahren nach einem der Ansprüche 1 bis 8, wobei die Umwandlung der Stufe a) mittels gasförmiger Salzsäure durchgeführt wird.

10. Verwendung von Ethanol zur Umwandlung von Amidin mit der Formel (III): 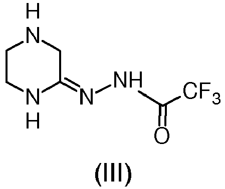 in Triazol mit der Formel (II) und Salz davon: 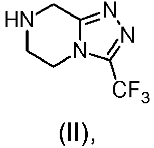 wodurch die Suspension der Verbindung (II) in Ethanol erhalten wird.

11. Verfahren zur Herstellung von Sitagliptin mit der Formel (I) und Salz davon: 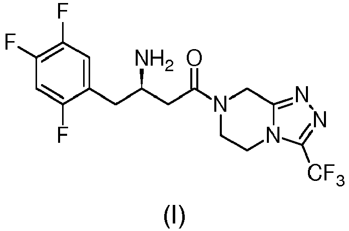 bestehend aus den folgenden Stufen:
A) Umwandlung von Amidin in Triazol gemäß dem Verfahren nach einem der Ansprüche 1 bis 9,
B) Umwandlung von Triazol aus der Stufe A), um Sitagliptin zu erhalten.

12. Verfahren nach Anspruch 11, wobei die Stufe B) mithilfe der folgenden Stufen durchgeführt wird:
C) Umwandlung von Triazol in Ketoamid mit der Formel (IV): 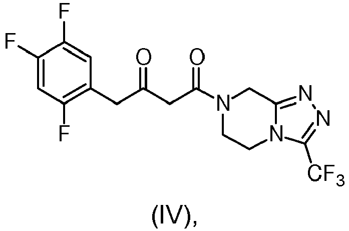
D) Umsetzung zur Produktion von Enaminamid mit der Formel (V) aus Ketoamid, das in Stufe C) produziert wurde, 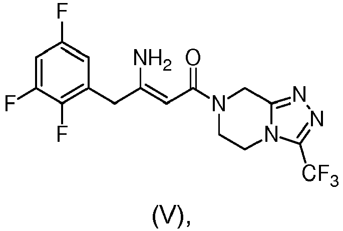
E) Umwandlung von Enaminamid aus Stufe D) in Sitagliptin mit der Formel (I): 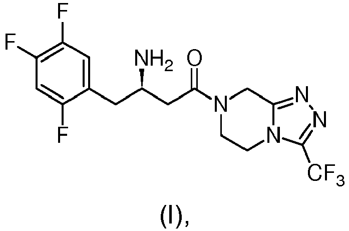 oder mittels des alternativen Verfahrens zum Erhalten von Sitagliptin, bestehend aus den folgenden Stufen:
C1) Umwandlung von Triazol in Ketoamid mit der Formel (IV): 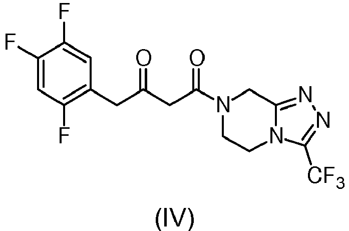
D1) enzymatische Umwandlung von Ketoamid mit der Formel (IV), erhalten in der vorhergehenden Stufe C1), in Sitagliptin mit der Formel (I):

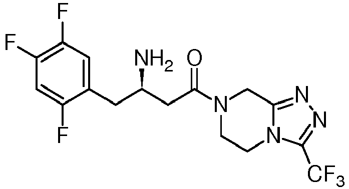

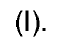

## Revendications

1. Procédé de préparation de Triazole de formule (II) et d'un sel de celui-ci: 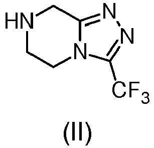 comprenant les étapes suivantes:
a) conversion dans un environnement acide d'une Amidine de formule (III): 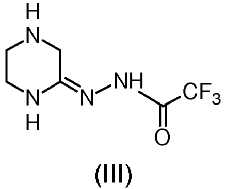 en un composé de formule (II), dans lequel la conversion est effectuée dans de l'éthanol,
b) obtention d'une suspension du composé (II) dans de l'éthanol, sans l'addition d'un quelconque autre solvant,
c) isolement du produit de formule (II) d'une suspension dudit composé dans de l'éthanol.

2. Procédé selon la revendication 1, dans lequel la conversion de l'étape a) est effectuée en dosant une solution d'acide chlorhydrique gazeux sur de l'alcool éthylique dénaturé à un mélange d'Amidine de formule (III) et d'éthanol.

3. Procédé selon l'une quelconque des revendications 1 à 2, dans lequel l'étape a) et/ou l'étape b) sont effectuées sans la présence d'eau.

4. Procédé selon l'une quelconque des revendications 1 à 2, dans lequel, dans l'étape a) ou dans l'une et l'autre des étapes étape a) et étape b), l'éthanol contient une quantité d'eau inférieure à 0.10 volume par comparaison avec le composé de formule (III).

5. Procédé selon l'une quelconque des revendications 1 à 4, dans lequel l'une et l'autre des étapes a) et b) sont effectuées avec une quantité de volumes d'éthanol comprise entre environ 1 et environ 6 par comparaison avec le composé de formule (III).

6. Procédé selon l'une quelconque des revendications 1 à 5, dans lequel les volumes d'éthanol sont compris entre 1.5 et 2.0 volumes par comparaison avec le composé de formule (III).

7. Procédé selon l'une quelconque des revendications 1 à 6, dans lequel les volumes d'éthanol sont de 1.7 volume par comparaison avec le composé de formule (III).

8. Procédé selon l'une quelconque des revendications 1 à 7, dans lequel le Triazole de formule (II) est sous forme de sel chlorhydrate.

9. Procédé selon l'une quelconque des revendications 1 à 8, dans lequel la conversion de l'étape a) est effectuée au moyen d'acide chlorhydrique gazeux.

10. Utilisation d'éthanol pour la conversion d'Amidine de formule (III): 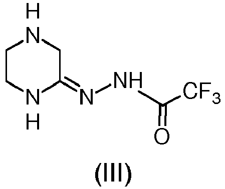 en Triazole de formule (II) et un sel de celui-ci: 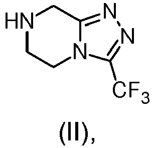 en obtenant ainsi une suspension de composé (II) dans de l'éthanol.

11. Procédé de préparation de Sitagliptine de formule (I) et de sels de celle-ci: 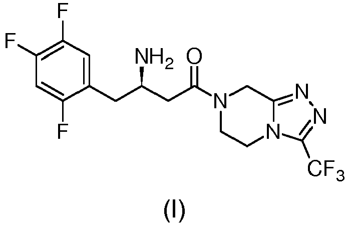 constitué des étapes suivantes:
A) conversion d'Amidine en Triazole selon le procédé d'une quelconque des revendications 1 à 9,
B) conversion du Triazole de l'étape A) pour obtenir la Sitagliptine.

12. Procédé selon la revendication 11, dans lequel l'étape B) est effectuée au moyen des étapes suivantes:
C) la conversion de Triazole en Cétoamide de formule (IV): 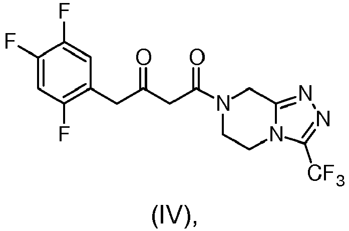
D) une réaction pour produire un Énamine Amide de formule (V) à partir de Cétoamide, produit à l'étape C) 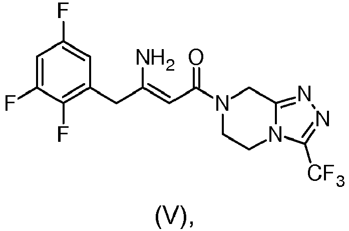
E) la conversion de l'Énamine Amide de l'étape D) en Sitagliptine de formule (I): 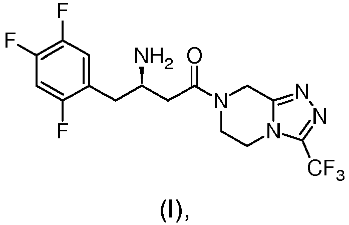 ou au moyen du procédé alternatif pour obtenir de la Sitagliptine, constitué des étapes suivantes:
C1) la conversion de Triazole en Cétoamide de formule (IV): 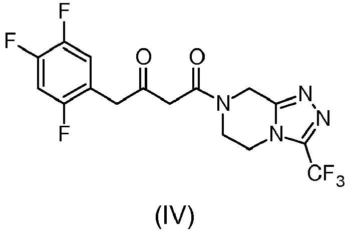
D1) la conversion enzymatique de Cétoamide de formule (IV), obtenu par l'étape C1) précédente, en Sitagliptine de formule (I):
